# EUROPEAN PATENT APPLICATION

(11) **EP 1 994 925 A1**
(43) Date of publication of application: **26.11.2008**
(21) Application number: 07712569.8
(22) Date of filing: 18.01.2007
(51) Int. Cl.: A61K 9/19, A61K 31/337

(54) **TAXANE PHARMACEUTICAL FORMULATION, SOLID TAXANE COMPOSITION, METHOD FOR PREPARING THE SOLID TAXANE COMPOSITION, COMPOSITION FOR SOLUBILISING SAID SOLID TAXANE COMPOSITION AND SET OF ELEMENTS (KIT) FOR THE INJECTABLE TAXANE FORMULATION**

(30) Priority: 20.01.2006 AR P060100208
(71) Applicant: Eriochem, S.A., Parana, Entre Rios (AR)
(72) Inventor: BOUZADA, Antonio Osvaldo, E3102FLD Paraná (AR); NUÑEZ, José Lucio, E3100FGD Paraná (AR); ITURRASPE, José Bernardo, S3002FOE Santa Fe (AR); MOYANO DE ITURRASPE, Nora Adriana, S3002FOE Santa Fe (AR)
(74) Representative: Agasse, Stéphane
(86) International application number: PCT/ES2007/070012
(87) International publication number: WO 2007/082978

(57) **Abstract**

A pharmaceutical formulation of taxane intended to be administered to mammals, preferably humans, comprising two compositions combined prior to being administered, forming a transparent solution free from precipitates, in which said compositions comprise a solid composition of lyophilised taxane, free from tensoactives, oils, polymers, solubility enhancers, preservatives and excipients and a solubilising composition of said lyophilised taxane solid composition that comprises at least one tensoactive. Said formulation is free from polysorbate 80 and polyoxyethylated castor oil. A procedure for the preparation of said solid composition by means of the lyophilisation of taxane in a lyophilising organic solvent. A kit for said injectable formulation of taxane comprising a prefilled syringe. Also a pharmaceutical taxane solution for perfusion, free from organic solvent.

## Description

### Field of the invention

This invention belongs to the field of the formulations of pharmaceutical drugs which are poorly soluble in water. Particularly, it refers to oncological drug formulations, in which said drugs belong to the taxane group. More specifically, the invention is directed to formulations intended for parenteral infusion processes typical of oncological chemotherapy with docetaxel and paclitaxel.

### State of the art

The pharmacological formulations of drugs which are poorly soluble in aqueous media have been extensively studied over the last decades. Innumerable strategies have been developed in order to inject these drugs into mammals with the aim of improving their pharmacotechnical properties and ameliorate their side effects.

Concerning formulations which are suitable for the preparation of solutions for parenteral infusion over a long period, and especially for oncological chemotherapy treatments, technical problems arise, namely as how to maintain these drugs in the aqueous solution of the parenteral infusion for periods of at least 4 hours for conventional infusion protocols and at least for 72 hours for administration by means of a continuous infusion pump. Another problem described is the *in situ* gelification which occurs at the time of injecting the drug solution in the presence of high tensoactive concentrations, in the container of the parenteral infusion. This phenomenon is described as irreversible, for the tensoactive gelifies and does not solubilise in the parenteral solution.

Of a particular interest for this invention are the compounds of the taxane family, products extracted from the leaves and bark of a tree commonly known as European yew (*Taxus Baccata* and other species of the Taxus family) as paclitaxel, as well as semi-synthetic products obtained from baccatin III or from 10-deacetylbaccatin III which are also extracted from yew, like docetaxel. Those taxanes obtained from biotechnological processes are also of interest for the present invention.
The medical uses of taxanes are varied; their anti-tumoral effects can be mentioned among others. Some examples of cancer which can be treated with docetaxel are: locally advanced or metastatic cancer, breast cancer, non-small cell lung cancer, hormone-refractory prostate cancer and ovarian cancer, and gastric cancer.

Patent US4814470 to Colin, Michel et al., from the company Rhône-Poulenc Santé (Courbevoie, FR), refers to a taxane pharmaceutical composition from which 10-deacetylbaccatin III derivatives are obtained, specially docetaxel. This document describes a synthesis that ends with a crystallisation and a formulation comprising the dissolution of the crystals obtained in a mixture of equal parts of non-ionic tensoactives and alcohol. This formulation gelifies when injected into the parenteral infusion bag.

A series of patents from the company Rhône-Poulenc Santé (Courbevoie, FR) protects the current formulation of docetaxel (US5438072, US5698582, US5714512 and US5750561). This technology solves the *in situ* precipitation and gelification problems by a formulation having two solutions, one of taxane in polysorbate 80 and alcohol (which can be present in very low concentrations) and the other of water and ethanol. These documents describe a preparation process which consists of mixing both solutions without intense stirring, to generate a solution of Taxotere 10mg/ml (stable between 2 and 25°C for 4 hours), which solves the problem of gelification *in situ* by diluting such solution in an aqueous 5% dextrose solution or normal saline solution of sodium chloride at 0.9% to obtain the infusion solution at a concentration of 0.3 to 0.74mg/ml (stable for 8 hours, between 2 and 25°C).

This liquid formulation of docetaxel in polysorbate 80 (in the presence or absence of alcohol) poses the problem of stability in time; being a room temperature between 2 and 25°C recommended for its conservation. Besides, the presence of polysorbate 80 causes known adverse side effects due to the incorporation of said tensoactive in high concentrations -which are necessary to maintain the drug in solution- into the blood stream. The same happens with the formulation of paclitaxel available on the market, which requires high concentrations of Cremophor. The adverse side effects caused by these tensoactives present in taxane formulations currently available, particularly docetaxel and paclitaxel, require a pre-treatment with steroids or antihistamines before oncological chemotherapy as described in the literature, as follows: 'ten Tije AJ, Verweij J, Loos WJ, Sparreboom A. Pharmacological Effects of Formulation Vehicles: Implications for Cancer Chemotherapy. Clin Pharmacokinet 2003; 42: 665-685'; 'Rowinsky EK, Eisenhauer EA, Chaudhry V et al. Clinical toxicities encountered with paclitaxel (Taxol). Semin Oncol 1993, 20: 1-15'; 'Bernstein B. Docetaxel as an Alternative to Paclitaxel after Acute Hypersensitivity Reactions. Ann Pharmacother 2000; 34:1332-1335'; 'Novel Formulations of Taxanes: A review. Old Wine in a New Bottle? K. L. Hennenfentl & R. Govindan Annals of Oncology 17: 735-749, 2006 doi: 10.1093/annonc/mjd 100 Published on line 19 December, 2005'.

Likewise, the formulation of docetaxel currently available on the market requires for its use a process which involves several steps and a certain risk for the doctors and nurses involved in its administration. These steps, aimed at ensuring the proper administration of this drug means: extracting the solvent from an ampoule and introducing it into a vial containing docetaxel solution, gently agitating to homogenize the solution, allowing a rest time for foam to disappear, extracting the mix and injecting it into the perfusion bag or flask filled with saline solution or dextrose, homogenizing and finally inspecting it for possible precipitate formation before administering it to the patient (should precipitation appear, the solution must be disposed of with the resulting economical negative impact).

The whole process must be carried out aseptically. The risks of contamination are high and the operation requires trained personnel and a considerable time. There is also a risk of contamination for health care providers in contact with the cytotoxic solution handled, due to the aerosolisation of the drug.

Currently, there is a need to simplify such process of administration, to shorten the time for the preparation of the perfusion formulation and to decrease the risks implied.

The toxic effects of the tensoactives used in the formulation of docetaxel and paclitaxel, such as polyoxyethylated castor oil (Emulphor ® or Cremophor®) or polysorbate 80 (Tween 80 ®) are known. The pharmacological and biological effects caused by these tensoactives have been described as acute hypersensitivity reactions, peripheral neuropathy, cumulative fluid retention syndrome, etc. A great number of patients cannot be treated due to the side effects of such tensoactives, eg. patients presenting hypersensitivity, patients with impaired renal function, elderly patients, patients suffering from a cardiopathy, etc. These tensoactives also affect the availability of the drugs which are solubilised and administered intravenously.

That is the reason why great efforts have been made in the scientific field in order to find formulations which prevent or decrease the use of said tensoactives. Among the strategies described in the state of the art, we can mention the development of albumin nanoparticles, polyglutamates, taxane analogs, prodrugs, emulsions, liposomes, etc.

For the purpose of reference, literature is provided to offer a detailed description of the latest development and clinical tests: 'Novel formulations of taxanes: A Review. Old Wine in a New Bottle?' K. L. Hennenfentl & R.. Govidan 2 1st Louis college Pharmacy. Ortho Biotech Clinical Affairs, LLC, St Louis MO; 2 Alvin J Siteman Cancer Center, Washington University School of Medicine, St. Louis, MO, USA accepted 7 November, 2005. Other works contributing solutions to these problems are: 'Castro CA et al. Pharmacol Biochem Behav, 1995 Apr; 50(4):521-6; Sanzgiri UY et al., Fundam Appl Toxicol, 1997 Mar; 36(1):54-61; ten Tije AJ et al., Clin Pharmacokinet. 2003; 42(7):65-85':' Constantine JW et al., Experientia. 1979 Mar 15;35(3):338-9':' Van Zuylen L et al., Invest New Drugs 2001 may;19(2):125-41':' Bergh M et al., Contact Dermatitis. 1997 Jul; 37(1):9-18)'.

The WHO has estimated that the maximum daily dose of polysorbate 80 is 25mg/kg of body weight (FAO WHO. Tech. Rep. Ser. Wld. Hlth. Org. 1974, N° 539). Therefore, a man weighing 75 kg may be administered 1.875 g of polysorbate 80 per day; i.e., for a dosage form of 75 mg of Taxotere® per m² commonly used for lung or prostate cancer, a man who is 1.75m tall and weighs 75 kg, has a body surface of 1.90 m² and is to be administered 143 mg of docetaxel along with approximately 3.6 g of polysorbate 80 (40 mg of docetaxel per ml of Tween 80®), i.e. almost 2-fold the maximum daily dosage form of polysorbate 80 recommended by the WHO.

On the other hand, lyophilised formulations of certain injectable drugs tend to be more advantageous than injectable liquid formulations, particularly in those cases in which the lyophilised solution is of a higher chemical and physical stability, i.e, it has a longer shelf-life and is more resistant to higher temperatures, as those in the warm climates of the regions belonging to Zone 4, according to the International Committee for Harmonization (ICH).

Particularly, the process of lyophilisation of drugs which are poorly soluble in water, mainly taxanes, presents great difficulties because the standard techniques of lyophilisation consist of freezing aqueous solutions and subjecting them to vacuum to achieve sublimation. Apart from water there are not many solvents which allow this procedure within acceptable pharmacotechnical conditions. In order to be liophilised, a good formulation should not pose "puffing" problems, i.e, when frozen it should not generate a plastic solid which bubbles on sublimation. Furthermore, it should be a good heat conductor and the solvent should generate a lyophilised plug of suitable pharmacotechnical characteristics. Moreover, the material structure should meet the condition of having spaces to allow the diffusion of the gas produced by the sublimation of the solvent through the cake. Besides, the plug should be rigid enough to support its own structure, aided by an excipient if needed.

There have been many attempts in the state of the art in order to solve the problem of toxicity of non-ionic tensoactives, proposing taxane lyophilised formulations. For example, patent WO 99/24073 by Géczy, J (Thissen Laboratories S.A.) proposes to lyophilise docetaxel and paclitaxel starting from a hydroalcoholic solution of these drugs and cyclodextrins. This solution allows to obtain a liophilisate containing taxane complexed to a lyophilised powder cyclodextrin (it uses 2hydroxypropyl β-cyclodextrin for docetaxel in a mass ratio of approximately 1:100 of active principle: cyclodrextrin). This lyophilisate can be dissolved in an aqueous solution of up to 1mg/ml, ready to perfuse. Although this eliminates the use of non-ionic tensoactives, this formulation incorporates into the blood stream a complex between taxane and cyclodextrin which not only increases stability as regards precipitation of an oversaturated aqueous solution of taxane, but which can also modify the significant pharmacodynamic properties of taxane. Therefore, a toxicological and clinical study is required to endorse the use of this new complex between taxane and cyclodextrin. Besides, it involves complex elaboration processes.

Other attempts to obtain taxane lyophilisates are shown in the state of the art, such as the application for patent US20030099674 by Chen, Andrew in which obtaining a lyophilised taxane from an oil/water emulsion using lecithin as a tensoactive and sucrose as an anti-adhesion agent is proposed. Bile salts are among the surfactants mentioned in the description. Taxane is dissolved in ethanol and water, and the solvents are then eliminated by lyophilisation, generating a taxane liposome when it is reconstituted with water.

Furthermore, the application of patent US20030099675 by Jeong, Seo Young, proposes a liquid formulation having an organic solvent, an emulsifier and a monoglyceride. It also proposes forming an emulsion in water from a liquid formulation and lyophilising it. The use of active drugs such as paclitaxel is mentioned in this document.

In these last two applications a taxane lyophilisate with a good solubility in water is obtained. However, said taxane lyophilisate poses similar problems to those mentioned before as regards the use of taxane emulsions which can modify their pharmacodynamics. Naturally, emulsions and microemulsions as liposomes generate autoimmune responses when administered endovenously and are attacked by macrophages, which causes an important part of the dosage to be unavailable for the desired action apart from generally requiring a pretreatment with steroids or antihistamines.

Other technological development as the one described in the application US20030187062 by Zenoni Mauricio, et al. from ACS DOBFAR S.A. proposes obtaining micro or nanoparticles by lyophilising paclitaxel and albumin.

There is a vast literature describing taxane liposomes as in patent EP 1332755, in which a lyophilisate of paclitaxel is obtained using a compound like lecithin or cholesterol in a solution of isopropanol or ethanol in order to subsequently obtain liposomes which are always lyophilised from aqueous solutions. One of the drawbacks of these developments is that they modify the pharmacodynamics of taxane, as they have a short useful life and require a cold chain for their preservation, apart from generating an immune response and causing the attack of macrofages which decreases the effect of the drug considerably.

There are many patents claiming polymer micellae in the literature, namely patents US5543158 and US6322805. In particular, patent US6322805 refers to obtaining biodegradable polymeric micellae capable of solubilising hydrophobic drugs comprising amphiphillic block copolymers, which have a hydrophilic polyalkyl oxide and a biodegradable hydrophobic polymer selected from the group consisting of polylactic acid, polyglycolic, polylactic-co-glycolic acid, poly(epsilon-caprolactone) derivatives and the mixtures thereof. It describes how the hydrophobic drug is trapped in the micelle in the absence of a covalent bond. These micellae form a solution in water acting as solubilising agent. This solution can be lyophilised, preserved and reconstituted with water or isotonic solution. This patent does not solve the problem of increasing the solubility of taxane as such, but it presents a complex process of synthesis of a specific copolymer to generate taxane micellae. In spite of having done promising tests regarding the characteristics of stability, components are added to the drug thus radically changing not only the bioavailability but also the kinetics of the original taxane. This patent does not describe the method to obtain a sterile solution by means of reconstitution.

The document US Pat. N° 6780324 describes a process in which a solution of a biologically active hydrophobic agent is formed in combination with a dispersing agent and an organic solvent or even a mixture in which water can be included. This mixture can be lyophilised and redissolved to form a nanodispersion or a micelle solution. Drug is not lost during this process; it can be sterilized by filtration. A transparent solution is obtained by reconstitution but the lyophilisate has other components apart from the drug, thus posing risks not only for its chemical stability but also for its bioavalability. On the other hand, the procedure proposed requires operations such as sonication, intense stirring and heating to obtain a solution to be lyophilised which can either destroy micellae or cause taxane degradation.

Patent US6610317 B2 by Julie Straub et al. describes a porous matrix of paclitaxel produced by mixing taxane dissolved in organic solvent, specifically ethanol, with polysorbate 80, other tensoactives and excipients like mannitol to further evaporate the solvent by spray drying. This formulation proposes a soluble solid having components in its formulation which can cause side effects such as those generated by polysorbate 80. Besides, it proposes heating the drug for its drying, a step which originates degradation products known by the art. On the other hand, the paclitaxel solution that can be produced following the teachings of said patent contains 80% of ethanol, which would make a lyophilisation process impossible to be applied to generate the solid formulation proposed.

The application of patent US2004/0247624 A1 by Evan Charles Unger, et al. describes another method to formulate drugs poorly soluble in water. It proposes to elaborate a solid composition lyophilising a filtrate solution of an organic solvent, the poorly soluble drug and at least a stabilizing agent which does not have a covalent bond with the drug. The only example with paclitaxel (example #3) describes a complex process of sonication and heating of a mixture of two polymers in high concentration, t-buthanol and the drug until solubilisation is reached. Subsequently lyophilisation is proposed to obtain a powder. Finally, it describes redissolution of such powder with another tensoactive solution which finally cannot dissolve the whole solid as it states that there are visible particles. Moreover, heating the drug in the solution at 60°C causes paclitaxel degradation. The application US2005/0152979 by Marc Besman et al. claims a lyophilised composition of a drug poorly soluble in water which contains said drug, a polymer and an agent to improve reconstitution. In spite of claiming paclitaxel and docetaxel as drugs suitable to be used by the invention, the tests it states are performed with a paclitaxel conjugate named CT-2103 which is an ester conjugate of alpha-poly-(L)glutamic acid and paclitaxel, of a much higher solubility in aqueous solutions than paclitaxel itself. Neither paclitaxel nor docetaxel dissolves in sodium phosphate aqueous solutions with the excipients and tensoactives described in this document.

The application of patent US2003/0099674 A1 by Andrew Chen describes a taxane composition solubilisable in water elaborated by the lyophilisation of an emulsion of the drug in oil, also containing an anti-adhesion agent. An important degradation of taxanes in oils is observed in this patent, when they are subjected to a temperature of 60°C for a month. This technology poses the problems described above as regards stability and macrophage reactions in the presence of emulsions injected in the body of mammals.

Other technologies like those used in taxane conjugates are described in documents such as the application of patent US2003/0147807 by Chun Li et al. which characterises a taxane composition soluble in water. However, it refers to conjugates of paclitaxel and docetaxel attached to soluble polymers such as polyglutaminic acid, polyaspartic acid, etc.

Other patents evaluated as the state of the art related to the present invention were the following: US2005/0191323, WO9814174, US6630121, US6607784, WO2005/044225, US2006/0127420, US2006/0078619, US2004/0091541, US2003/0215496, US2001/0018072, US5922754 and WO2005025499.

The current state of the art offers a great number of solutions to obtain formulations of drugs of a low solubility in water. However, it was not possible to obtain in said solutions a solid composition of liophilised taxane free from other compounds, mainly from those which modify its pharmacodynamics and chemical stability during storage.

To achieve the solubilisation of these drugs of a poor solubility in aqueous media, a great number of methods have been proposed in the state of the prior art, such as intense agitation, heating, sonication, solvent evaporation, dialysis, spray-drying, emulsification/evaporation, micronisation, etc. The proposals involving lyophilisation as part of the procedure which have already been described generally start from a mixture containing organic solvents, water, polymers, excipients, tensoactives, lipids, and lipoproteins among other components. These mixtures usually involve complex emulsification or dilution processes that often require heating, sonication, intense stirring and use of polymers specially designed, among other procedures.

The present invention provides a pharmaceutical formulation of two components, one constituted by a taxane solid composition, especially lyophilised docetaxel and paclitaxel, and the other component constituted by a liquid solubilising composition.

Moreover, the present invention provides said solid composition of taxane, particularly docetaxel and paclitaxel, having extraordinary diluting features compared to pharmaceutical actives -either anhydrous or polyhydrated- available on the market. Said solid composition does not contain excipients, polymers or tensoactives, being free from any other component. This solid composition does not contain polyoxyethylated castor oil or polysorbate 80. Besides, it can be obtained by a-simple procedure as it does not require heating, sonication or intense agitation, it is easy to repeat and it only involves two components: the pharmaceutical active and an organic solvent for lyophilisation. The solid composition of the present invention is totally soluble in an aqueous tensoactive solution in less than a minute, free from the addition of an organic solvent.

The present invention provides an innovative method which allows lyophilisation from a taxane solution in an organic solvent where said solution is obtained without the need of external, mechanic or thermal media to achieve a rapid and total dissolution. Said solution is subjected to lyophilisation from which a liophilised cake is obtained, containing only said taxane and traces of the lyophilisation solvent. Thus, a pharmaceutical active of a great specific area and an extraordinarily enhanced solubility is obtained following a very simple procedure.

There are many taxane solubilisation methods in the prior art, but in all cases the active pharmaceutical ingredient (API) is dissolved in an organic solvent, generally ethanol and subsequently other components are added namely tensoactives, water, excipients, etc. No literature has been found so as to infer that paclitaxel and docetaxel are solubilised in an aqueous solution in the absence of an organic solvent added to it.

Furthermore, it has been proved that the addition of organic solvents such as ethanol enhances the solubility of taxanes but affects their stability favouring anticipated precipitation of the drug when formulated in an aqueous solution of perfusion.

The present invention also provides a kit which allows the use of the formulation safely, since it decreases the risks of contaminating the drug and health-care staff who manipulates it, while it also facilitates the operation of drug administration. Said kit comprises the two compositions mentioned contained in sterile compartments, isolated from one another. Said kit also provides a syringe.

In a preferred version of the invention said syringe is prefilled and comprises said sterile compartments. Thus, the preparation of the perfusion formulation is simplified as both compartments come into contact, and by means of gentle movements, the dissolution of the liophilised solid taxane composition is achieved in said solubilising composition to be finally injected into the perfusion bag or flask containing saline solution or dextrose.

This prefilled syringe makes the operation of preparating the perfusion solution extremely easy and decreases execution time remarkably by eliminating to a considerable extent the risks of contamination either for the products or the operators and patients, which occurs with the product currently available.

The formulation of the invention enables the elimination of the habitual antihistamine pretreatment. This is due to the fact that this formulation is free from polysorbate 80 and polyoxyethylated castor oil. Furthermore, this formulation allows its administration in shorter times than the usual ones indicated in the practice, as long perfusion periods are necessary due to the presence of polysorbate 80. Thus, periods of less than 30 minutes would be necessary for the administration of taxanes using the formulation of the present invention.

Both, decreasing operative times for the preparation of the perfusion solution and, decreasing the perfusion times during the administration of the formulation of the invention provide the following benefits: a greater number of patients being treated in day care hospitals in the oncological clinical service of the current health system (either public or private), reduced operative costs on the basis of pharmacoeconomics, due to a better utilization of sanitary resources and prioritization of the safety of health-care providers involved in said manipulation (the risk of the stock solution which comes into contact with skin or mucosa of health-care providers is reduced to almost 0).

As a great number of adverse side effects is attributed to polysorbate 80, this new formulation can be administered to patients who cannot receive it today, namely patients with renal illnesses, cardiopathies, the elderly, and those with polysorbate 80 hypersensitivity, etc. It also can be administered to patients suffering from kidney cancer as it does not contain polysorbate 80 which causes fluid retention syndrome.

### Summary of the invention

The first object of the present invention is to solve the problems described in the prior state of the art and to provide a pharmaceutical formulation of taxane having an enhanced stability allowing storage at ambient temperature in tropical and subtropical climates, which facilitates the operations for the infusion preparation, is free from tensoactives and toxic excipients, particularly free from polyoxyethylated castor oil and polysorbate 80. Said formulation is intended for treatment in patients with hypersensitivity to said tensoactives, patients suffering from renal illnesses such as saline retention syndrome, the elderly and patients with cardiopathies, etc. This formulation comprises a solid composition of said lyophilised taxane and a solubilising composition of said solid composition. In particular, it is directed to an injectable formulation of a taxane such as paclitaxel or docetaxel, suitable for use in parenteral infusion solutions in mammals, preferably humans.
A second object of the present invention is to provide said solid composition of a taxane, suitable to prepare pharmaceutical formulations of a great stability even at high temperatures (60°C) as it is in solid state, essentially free from other components, of an enhanced solubility due to its high specific area and to its low apparent density, which can be solubilised by means of an aqueous solution of a tensoactive in the absence of an organic solvent. This solid composition comprises a lyophilisate from a solution of at least said taxane in an organic solvent.

A third object of the present invention is to provide said solubilising composition of said lyophilised taxane solid composition suitable to be injected into parenteral infusion solutions comprising at least a polymeric tensoactive of a low toxicity.

A forth object of the present invention is a procedure to prepare said lyophilised taxane solid composition comprising the following steps:
a) dissolving said taxane in lyophilisation organic solvent
b) lyophilising
c) optionally, drying
This procedure, in its preferred embodiment, also involves a sterilisation step. Said sterilisation step is preferably performed by means of a sterilising filtration of the solution obtained in step a).

A fifth object of the present invention, a pharmaceutical perfusion solution, contains less than 1 mg/ml taxane in normal saline solution or dextrose solution and it also only contains Solutol(R), essentially free from organic solvent, other tensoactives, oils, other polymers, solubility enhancers, preservatives and excipients.

A sixth object of the present invention is to provide kit comprising: a first container holding said solid composition of taxane, a second container holding said solubilising composition of said solid composition of taxane and a syringe.

A seventh object of the present invention is a prefilled syringe comprising said containers.

### Brief description of the invention

The pharmaceutical formulation of taxane, main object of this present invention, to be administered to mammals, mainly humans, preferably free from polysorbate 80 and free from polyethoxilated, comprises two compositions which are combined prior to its administration forming a transparent solution free from precipitates, where said compositions comprise: a solid composition of lyophilised taxane, preferably free from tensoactives, oils, polymers, solubility enhancers, preservatives and excipients; and a solubilising composition of said solid composition of lyophilised taxane comprising at least one tensoactive. Said solid composition presents an apparent density lower than 0.1g/ml, preferably from 0.004 g/ml and 0.05 g/ml, most preferably from 0.006 g/ml and 0.02 g/ml. Said solid composition is soluble in an aqueous solution of Solutol ® HS 15 to 20% in less than a minute and in the absence of an added organic solvent. Furthermore, said solid composition is chemically stable at 60°C for at least 28 days with a degradation of less than 1%. Moreover, said solid composition of the invention is obtainable by the lyophilisation of a solution comprising a lyophilisation organic solvent, selected from the group comprised by dioxane, acetic acid, dimethylsulphoxide or a mixture thereof, preferably dioxane or acetic acid, and a taxane at a concentration from 0.1 to 50% preferably 0.1 and 6%, preferably in the absence of tensoactives, oils, polymers, solubility enhancers, preservatives and excipients. Also, said solid composition has a residual concentration of lyophilisation organic solvent lower than 8%, preferably less than 3%. Said taxane is selected from the group comprised by derivatives of baccatine III, 10-deacethilbaccatine III and the conjugates, salts, hydrates and solvates thereof, preferably docetaxel and paclitaxel. Said solubilising composition comprises a polymeric tensoactive at a concentration from 1% to 100%, preferably from 5% to 40% and water in the absence of organic solvents. Said tensoactive is polymeric and selected from the set comprised by macrogol hydroxystearate such as Solutol®, poloxamer such as Lutrol® and polyvynilpyrrolidone or a mixture thereof. A preferred solubilising composition of the invention comprises Solutol®HS 15 from 10% to 50% and water from 50% to 90% (P/P%). Said solubilising composition dissolves said solid composition at a concentration of at least 4mg/ml in the absence of precipitates for at least 2 hours.

The procedure for the preparation of said solid composition of taxane suitable to prepare pharmaceutical formulations for mammals, particularly humans, another object of the invention, comprises the following steps:
a) dissolving said taxane in organic solvent, in the absence of polymers, tensoactives, oils or excipients
b) sterilising
c) lyophilising
d) optionally, drying

A pharmaceutical perfusion solution, another object of the present invention contains at least 1 mg/ml of taxane in a normal saline solution or dextrose solution and, it contains only Solutol®, essentially free from organic solvents, other tensoactives, oils, other polymers, solubility enhancers, preservatives and excipients.

A kit of elements, a further object of the present invention, comprises a first container holding said lyophilised taxane solid composition, a second container holding said solubilising composition of said taxane solid composition and a syringe. Preferably, said syringe is prefilled and comprises said first container and said second container.

A kit for the preparation of the injectable taxane formulation suitable to prepare parenteral infusion solutions for mammals, preferably humans, another object of the present invention, comprises a said taxane lyophilised solid composition; a solubilising composition of said solid composition of said taxane; a syringe that allows mixing said solubilising composition with said solid composition of said taxane and obtaining a transparent and stable solution of taxane at a concentration of at least 4mg/ml to be injected in a bag of parenteral infusion free from precipitation for at least 2 hours.

### Detailed description of the invention

As a result of thorough research and a great number of laboratory tests, a new pharmaceutical formulation of drugs with poor solubility in water, particularly taxanes, has been developed. Said invention achieves the preservation of the active substances over long periods, maintaining its stability during its useful life with no need of being kept in cold chain for preservation, even in tropical or subtropical countries. This new formulation eliminates the common problems encountered today with some of the formulations of the state of the art concerning *in situ* precipitation and gelification occurring upon injection into the perfusion flasks or bags containing a saline or dextrose solution. In chemotherapy treatments by blood perfusion, this new formulation allows said taxane to be introduced into the body of mammals, especially humans, in the absence of components which modify its solubility or bioavailability, or form a complex or join the drug covalently. Moreover, the formulation of the invention is a sterile formulation by means of a sterilising filtration procedure. This new pharmaceutical formulation of taxanes, which is the main object of the present invention, comprises both a solid composition of said taxane, lyophilised from a solution of organic solvents of lyophilisation, as well as a solubilising composition of said solid composition. An essential feature of this invention is that said solid composition of taxane is prepared by the lyophilisation of a solution of said taxane in a lyophilisation organic solvent. Said lyophilisation organic solvents have a relatively high fusion point (above -10°C) to allow solidification and further lyophilisation, but lower than 25°C to allow its work as a liquid at ambient temperature. Among others, acetic acid, dioxane and dimethylsulphoxide can be mentioned as appropriate lyophilisation organic solvents which allow an easy and fast dissolution of taxane without having to heat, sonicate or agitate energetically. Taxanes are soluble in these lyophilisation organic solvents in a wide range of concentrations of up to 50% p/p without precipitation during prolonged periods of time as a chemically and physically stable solution is produced. A mixture of these solvents can be used to realise the present invention.

Taxane solutions in such lyophilisation organic solvents allow, in its elaboration process, a sterilizing filtration by means of 0.2 µ filter. Said filtration is performed prior to lyophilisation to yield a sterile powder as the solid composition of the invention. The solubilising composition is also sterilized by filtration. Thus, solid sterilisation is avoided, which involves complex and expensive procedures and poses risks to the drug.

Besides, these taxane solutions in organic solvents of lyophilisation can be dosed in the liquid form in the container to be lyophilised, allowing to obtain from 5 to 200 mg per container, ready to be reconstituted before injection. The solid lyophilised composition resulting from these taxane solutions in organic solvent of lyophilisation is a powder with a large specific surface area which enables a complete and very rapid dissolution in the solubilising composition. Thus, a solid composition is obtained consisting in a liophylised cake or block of a good stability through time under high temperatures, above 25°C, remaining in a proper state without the need of cold chain. The stability tests performed, as shown in Example 6, proved that the solid composition of the invention withstands high temperatures, on the order of 60°C, for periods of at least a month with less than 1% degradation.

In addition, it has been proved that it is possible to use lyophilisation excipients normally used in the art, such as mannitol, lactose, bile acids, gelatin, etc. The lyophilisation excipient may be added to the taxane solution in lyophilisation organic solvent, either in powder form or as an aqueous solution. These taxane solutions in lyophilisation organic solvent withstand the addition of water up to a concentration of 30%.

Although it is possible to add water, lyophilisation excipients or an acid (such as citric, lactic, tartaric, ascorbic, acetic, hydrochloric acid or a mixture thereof), neither addition is essential to achieve the object of the present invention. Therefore, in a preferred embodiment of the present invention, only the taxane solution is lyophilised in organic solvent of lyophilisation, without any type of additives. This allows obtaining a solid composition of taxane of a large specific area, low apparent density and a taxane which is free from any interactions with other components.

Lyophilisation in vials allows obtaining an adequate lyophilisate in organic solvent for lyophilisation in concentrations from 6 mg/ml to 200 mg/ml.

The apparent density of the lyophilisate is defined as the quotient between the mass of the lyophilisation cake in grams and its volume in millilitres. This variable has been carefully evaluated and after innumerable experiences the values of apparent density were obtained, in which the technical effects of the invention are possible, such as the easy reconstitution of said lyophilised taxane solid composition in an aqueous solution free from organic solvent. It has been proved that the solubility of the solid composition of the invention improves as its apparent density decreases. Therefore, the lower the apparent density of the lyophilisation cake, the faster it dissolves. This effect can be observed in the results of Example 4. Likewise, if the apparent density is too low, the size of the container required is incompatible with sanitary manipulation.

These values of apparent density are lower than 0.1 gm/ml preferably between 0.004 gm/ml and 0.05 gm/ml, more preferably between 0.006 gm/ml and 0.02 gm/ml.

The content of residual solvent in the lyophilisate is normally lower than 8%, preferably below 3%.

In a lyophilisation process, with secondary drying stages of 24 hours, the quantity amount) of residual solvent is no greater than 8% for acetic acid and 3% for dioxane. These values can be reduced up to 3 and 1% increasing drying temperature up to 50°C and extending the drying time for another 24/48 hours, without major problems of active drug degradation.

Laboratory tests have proved that the pharmaceutical active available in the market, particularly trihydrate or anhydrous docetaxel, cannot be dissolved directly neither in a tensoactive aqueous solution, nor in pure polysorbate 80, whereas the solid composition of the invention is easily soluble in an aqueous solution of Solutol ® SH15 as well as in pure polysorbate 80. Also, it has been proved that said solid composition of the invention is rapidly soluble in a solution of polysorbate 80 (PS80): EtOH:water (25:9,75:65,25); unlike anhydrous docetaxel (API) available in the market.

An advantage of the solid composition of the invention is the remarkable improvement in the readiness to dissolve in solvents of the tensoactive and polymer group. Thus, said lyophilised solid composition can be easily dissolved in mixtures of Lutrol® F68, Lutrol® E400, Solutol® HS 15, avoiding the use of polysorbate 80.

Among the objects of the present invention it can be stated the elimination of polysorbate 80, polyoxyethylated castor oil and organic solvents such as ethanol, present in the current market formulations of docetaxel and paclitaxel.

The solid composition of the invention, obtainable by lyophilisation of taxanes in a solution of organic solvents of lyophilisation, allows the solubilisation of said taxanes rapidly not only in tensoactive aqueous solutions but also in pure tensoactives, such as polysorbate 80, in the absence of organic solvents added. It also allows the solubilisation in aqueous solutions of polymers such as Solutol® HS15, Lutrol ® F68, povidone, Lutrol® E400 and a mixture thereof. These formulations can be completely free from ethanol, mainly from polysorbates and polyoxyethylated castor oil.

In the state of the art, a usual way of accelerating the solubilisation process of taxanes is the use of organic solvents such as ethanol. This is the alternative mostly used in the abundant literature addressing this technological field. In a fist step, taxane is dissolved in the solvent and in a second stage the amphiphilic polymers, surfactant or tensoactives and the mixture thereof are added. The disadvantage of this alternative is the residual presence of solvent used and the lower stability of the solutions of the taxanes, especially docetaxel in the presence of ethanol. Besides, long periods of solubilisation, sonication, intense agitation, temperature increase and other operations are needed to achieve a stable solution or dispersion. Nevertheless, the use of an organic solvent, which is not necessary for the present invention, can be utilized as an ingredient of said solubilising composition.

The tensoactives suitable to be used in the present invention to formulate said solubilising composition, among others, can be: polyetylglycol, polyvinylpirrolidone, poloxamer, hydroxypropylcellulose, polymethacritates, polysine, poly vynil alcohol, poly acrylic acid, ethylene polyoxide, hyaluronic acid, dextrane sulphate and its derivatives, calcium stearate, glyceron monostearate., cetoestearilic alcohol, emulsifying wax of cetomacrogol, sorbitan esters, alquilic eter of ethylene polyoxide, macrogol esters, as cetomacrogol 1000, derivates of ricine oil polyocyothylenic, polysorbates, polysorbate 80, fat acid esters of polyoxyethylenesorbitane (TWEEN), estearates of polyoxyothylene, sodic dodecilsulphate, calcium carboxymetylcellulose, sodium carboxymetylcellulose, methylcellulose phtalate of hydroxipropylmetylcellulose, non crystalline cellulose, Triton.

A preferred embodiment of the present invention comprises as tensoactives for said solubilising composition Solutol® HS 15 (Polyethylene glycol 15-hydroxyestearate), a macrogol hydroxiestearate, Lutrol® F68, a poloxamer provided by the firm BASF, polyvynilpirrolidone, or mixtures thereof. In particular, Solutol® HS 15, Lutrol® F68 or the mixtures thereof are preferred.

Solutrol® HS 15 is a hydroxystearate macrogol. It is also known by the following names: polyethylene glycol-15-hydroxystearate, polyethylene glycol 660-12-hydrostearate, macrogol-15-hydroxystearate, CAS N°70142-34-6 is a polymeric tensoactive used in injectables to solubilise hydrophobic actives and avoid sedimentation and recrystallisation. Its low toxicity and extraordinary solubilising power, allow its use in high concentrations. A very low histaminic release has been proved after the administration to mammals as compared to polysorbates (application of Solutol® HS 15-A Potent Solubiliser with a Low Toxicity' F. Ruchatz). Some studies suggest that this solubiliser can present as desired side effect the reversion of multiple resistance of some carcinogenic cells as regards anticancer drugs. (K. H. Frömming et. al., Acta Pharm. Technol. 36(4). 1990, 214-220; J.S. Coon et. al., Cancer Res. 51 (3). 1991, 897-902; J.S. Coon, Proc. Am. Assoc. Cancer Res. 33.1992, 484; D. Hoover et. al., Fundam. Appl. Toxicol. 14 (1990), 589 pp.

The procedure for the preparation of the solid composition of taxane of the present invention comprises the following steps:
a) dissolving said taxane in organic solvent of lyophilisation in the absence of polymers, tensoactives, oils or excipients.
b) lyophilising
c) drying - optional

This procedure includes, in its preferred embodiment, a sterilisation which is preferably performed by sterilizing filtration of the solution obtained in step a). Said sterilising filtration is done with filtration membranes of materials which are inert to the organic solvents of lyophilisation used in the present invention. Among the filtrating membranes suitable to be used are those of Teflon or nylon, being the preferred pore size 0.22 µm. Other sterilisation methods such as gamma radiation, UV, etc. can be used.
Step a) of the procedure of the present invention is very simple, since it does not need energetic agitation, sonication, heating, additionof other solvents or another device used in the state of the art to achieve the solubilisation of taxanes. Once the taxane solution in organic solvent of lyophilisation is obtained, it is dosed in vials to lyophilise. This step is also simpler and faster than the ones already known in the state of the art as the solution in lyophilisation organic solvent is much less viscose (1.2caps) than the conventional polysorbate solutions (viscosity: around 400 cps). Therefore, not only production is simplified, but also production times are shortened applying the procedure of the present invention.

The variables involved in the operation of lyophilisation, such as time, temperature, pressure, etc. can adopt a wide range of values.

Different modes to carry out this operation successfully are known in the state of the art. A reference is made of a possible particular manner to perform this operation, as it was done in the practice by the inventors, for descriptive purposes only:

After powder taxane is dissolved in the organic solvent of lyophilisation, the solution is frozen in the container in which lyophilisation is to be done; the frozen product is allowed to mature to improve its properties; lyophilisation is performed at a tray temperature near the fusion point of the frozen product, which is generally reached at
-20 to 20°C, a condensation temperature which allows sublimation of vapour into a solid, generally from -40°C to -100°C and a pressure in the condenser which is lower than the vapour pressure of the solvent in the lyophilisation chamber.

Secondary drying is done at a tray temperature between 20 and 50°C, more especially between 25°C and 35°C and a total pressure lower than 1mmHg, for not less than 8 hours.

In a preferred embodiment of the present invention, said formulation is prepared starting from a solid composition of lyophilised taxane, in a sterilised container and a solubilising composition of Solutol® HS 15 in water, which after being mixed with one another, is injected into perfusion solution, for example normal saline solution or dextrose solution, to obtain a taxane solution stable for more than 2 hours, that is infused to patients undergoing oncological treatment.

The present invention also comprises a pharmaceutical perfusion solution containing less than 1mg/ml of taxane in normal saline solution or dextrose solution and also contains only Solutol®, essentially free from organic solvents, other tensoactives, oils, other polymers, solubility enhancers, preservatives and excipients. This perfusion solution is the one prepared with the pharmaceutical formulation of taxane of the present invention. Essentially, free from organic solvents means that it does not have the addition of organic solvents such as ethanol to enhance solubility, and it can only contain small concentrations of lyophilisation organic solvents which can remain from the process of preparation of the solid composition of the present invention. Besides, said pharmaceutical perfusion solution for the infusion of taxane in mammals, especially humans, for the treatment of cancer, is of a low toxicity, requiring shorter times for the perfusion process (less than 30 minutes) and does not require pretreatment with steroids or antihistamines since it is free from polysorbate 80, polyoxyethylated castor oil, emulsions or any other component.

Another preferred embodiment of the present invention comprises a kit consisting of a first container holding the solid composition of lyophilised taxane of the invention; a second container holding the solubilising composition of the invention and a syringe.

In another preferred embodiment of the present invention said syringe is prefilled and contains said containers, which are independent from one another, with means for connecting said containers prior to the administration and, optionally, with a filter.

It is worth highlighting that other drugs which are susceptible of being used as active matter in the formulation of the present invention are: albuterol, adapalene, doxazosin mesylate, mometasone furoate, ursodiol, amphotericin, enalapril maleate, felodipine, nefazodone hydrochloride, valrubicin, albendazole, conjugated estrogens, medroxyprogesterone acetate, nicardipine hydrochloride, zolpidem tartrate, amlodipine besylate, ethinyl estradiol, omeprazole, rubitecan, amlodipine besylate/benazepril hydrochloride, etodolac, paroxetine hydrochloride, atovaquone, felodipine, podofilox, paricalcitol, betamethasone dipropionate, fentanyl, pramipexole dihydrochloride, Vitamin D.sub.3 and related analogues, finasteride, quetiapine fumarate, alprostadil candesartan, cilexetil, fluconazole, ritonavir, busulfan, carbamazepine, flumazenil, risperidone, carbemazepine, carbidopa/levodopa, ganciclovir, saquinavir, amprenavir, carboplatin, glyburide, sertraline hydrochloride, rofecoxib carvedilol, halobetasolproprionate, sildenafil citrate, celecoxib, chlorthalidone, imiquimod, simvastatin, citalopram, ciprofloxacin, irinotecan hydrochloride, sparfloxacin, efavirenz, cisapride monohydrate, lansoprazole, tamsulosin hydrochloride, mofafinil, azithromycin, clarithromycin, letrozole, terbinafine hydrochloride, rosiglitazone maleate, diclofenac sodium, lomefloxacin hydrochloride, tirofiban hydrochloride, telmisartan, diazapam, loratadine, toremifene citrate, thalidomide, dinoprostone, mefloquine hydrochloride, trandolapril, docetaxel, mitoxantrone hydrochloride, tretinoin, etodolac, triamcinolone acetate, estradiol, ursodiol, nelfinavir mesylate, indinavir, beclomethasone dipropionate, oxaprozin, flutamide, famotidine, nifedipine, prednisone, cefuroxime, lorazepam, digoxin, lovastatin, griseofulvin, naproxen, ibuprofen, isotretinoin, tamoxifen citrate, nimodipine, amiodarone, and alprazolam, amphotericin B, cyclosporine, etoposide, topotecan, melphalane, idarubicine, doxorubicin, vinorelbine,vinblastine, vinchristine.

Reference is made herein to concentrations weight/weight (w/w) when no explicit reference is made to any other type of magnitudes.

For a better understanding of the technical and functional aspects of the present invention, and without implying a restriction on the scope of this patent application, there follows a set of examples of application involving some of the alternatives comprised in the present invention and a set of comparative examples to assess the differences stated in the light of the prior technique.

### EXAMPLES

Materials: In the experiments glass vials type I, 20mm- wide mouth, 22mm body diameter, 27 mm of height, Nuova Ompi, with a nominal capacity of 7 ml were used.
The 20 mm apyrogen caps for lyophilisation were sterilized with butyl-bromide from Helvoet Pharma.

The solvents used were quality, Acetic acid, Merk, item 1.000632511; Dioxane TEDIA, item DR-0480; ethanol, Merck, item 1.009832511. Distilled water was water quality for injectables (WFI) according to USP and EP specifications. Solutol® HS 15 BASF Trade Mark, item N° 51633963; Lutrol® F68 (Poloxamer 188) BASF, item N° 51633115; Lutrol® E400 BASF, item N° 51632267.

The lyophilisation assays were carried out in a VIRTIS ADVANTAGE lyophiliser driven by a MENTOR unit. The determinations of HPLC were performed either in a HPLC BECKMAN System Gold with solvent module model 118, diodes detector Model 116 and autoinjector Wilson model 234, or in a HPLC Waters model 1525 with binary pumps and a diode array detector, model 2996, a forced circulation oven Alltech (column thermostat Jetstream 2 Plus) and automatic sampler Waters 717 Plus. The solvent content was analyzed by gaseous chromatography AGILENT TECHNOLOGIES 6890N GC system with injector AGILENT TECHNOLOGIES 7683 B series with a sampler PERKIN ELMER Head Space Turbo Matrix 40 and a Mass Selective Detector AGILENT TECHNOLOGIES 5973 Network Mass selective Detector.

### Example 1

Docetaxel in a quantity of 389 mg was dissolved in glacial acetic acid (previously added with 1% water and kept at 100°C for 1 hour to hydrolyse all the acetic anhydrous present) to obtain 7.78 ml of solution (5% w/v). 0.20 ml were dosed (to obtain 10 mg of docetaxel per 7 ml flask; it was frozen at -18°C for 10 hours, and lyophilised. A solid composition of docetaxel formed by a lyophilised powder in the form of a cake was obtained.

### Example 2

Preparation of docetaxel lyophilisates in acetic acid: anhydrous docetaxel solutions were prepared by direct dilution in acetic acid to obtain solutions of concentrations of 50mg/ml, 40 mg/ml, 20mg/ml, 13.3 mg/ml, 10 mg/ml. These solutions were dosed in vials to obtain individual doses of 20 mg of docetaxel in each case. Thus, 0.4my, 0.5ml, 1 ml, 1.5 ml and 2 ml were dosed respectively of each concentration and were lyophilised to obtain a 20 mg/vial of docetaxel with a content of acetic acid lower than 3% and an apparent density of: 0.05, 0.04, 0.02, 0.013 and 0.01 respectively.

### Example 3

Preparation of docetaxel lyophilisates in dioxane: In the same way solutions of docetaxel were prepared in concentrations of 13.3 mg/ml and 10 mg/ml and were dosed 1.5 ml and 2 ml to obtain 20 mg of docetaxel in each vial with a dioxane content lower than 3% and an apparent density of 0.013 and 0.01 respectively.

### Example 4

Reconstitution or solubilisation assays were performed with the solid lyophilised compositions obtained in Examples 2 and 3. The vials containing 20 mg of docetaxel each, obtained in said examples were added with a solubilising composition formed by polysorbate 80:ethanol:water (25:9.75:65.25) and the time each test needed to completely solubilise the solid composition and result in a transparent liquid without precipitates was observed. The results are shown in the following table:

**TABLE 1**

| Lyophilising organic solvent | Apparent density of the cake | Volume of solubilising composition | Disolution reconstitution time |
|---|---|---|---|
| Acetic acid | 0.050 | 2 ml | 12 min |
| Acetic acid | 0.020 | 2 ml | 2 min |
| Acetic acid | 0.013 | 2 ml | 1 min |
| Dioxane | 0.013 | 2 ml | 1 min |
| Dioxane | 0.010 | 2 ml | <1 min |

The same assay was performed with 20mg of commercial (anhydrous) docetaxel and it was not possible to dilute it, not even with intense agitation, remaining thus in solid state.

### Example 5

Anhydrous docetaxel (1.02 g), purity 98.2%, was placed in a 250 ml container and dissolved in dioxane to reach a final volume of 100ml. Once dissolved, 2 ml of this solution were dosed in 7 ml vials, which were pre-covered and lyophilised following a lyophilisation cycle with freezing stages of -60°C for 240 min, and lyophilisation at -3 °C for 1500 min, at 100°C for 1500 min and final drying at 30°C for 2420 min. 48 samples of 20mg docetaxel were obtained. Each sample appeared as a lyophilisate of a homogeneous aspect without adherence to the walls, had an apparent density of 0.01 g/ml and was easily soluble in the following solvent mixtures:

**TABLE II**

| Solubilising composition | Volume of solubilising composition | Dissolution time |
|---|---|---|
| 1st- Solutol H S 15: water (25%) | 2 ml | < 1min |
| 2nd -PS80:EtOH:water (25:9.75:65.25) | 2 ml | < 1 min |
| 3rd-PS80: Lutrol E400:water (5:20:75) | 2 ml | < 2 min |
| 4th- PS80: Lutrol F68: EtOH: Water (12.5:12.5:10:65) | 2 ml | < 2 min |
| 5th - PS80:Solutol HS15:EtOH:Water (12,5:12,5:10:65) | 2 ml | < 1 min |
| 6th solutol HS 15:Lutrol F68: water (10:2:88) | 2 ml | <1 min |

In all these solubilising compositions except the second, less polysorbate was used than in the formulation currently available. In the third, 5 times less quantity was used and in the fourth and fifth only half the quantity was used. The solubilising composition of the 1^{st} and 6^{th} are the ones preferred in the present invention as they are free from polysorbate 80.

### Example 6

Stability tests on the solid composition of the invention were conducted at 60°C using docetaxel as taxane, and a comparison was made with docetaxel in a solution of polysorbate 80 available on the market. The purity of docetaxel was measured using HPLC. Purity results of docetaxel measured as a percentage of the peak area are shown in the following table.

**TABLE III**

| **Product** | **T=0 days** | **T=3 days** | **T=7 days** | **T=14 days** | **T=21 days** | **T=28 days** |
|---|---|---|---|---|---|---|
| **Lyo docetaxel - acetic. acid** | 99.24 | 99.21 | 99.08 | 98.94 | 98.68 | 98.37 |
| **Lyo docetaxel- Dioxane** | 99.54 | 99.35 | 99.04 | 98.27 | 98.65 | Not measured |
| **Docetaxel in PS 80** | 98.8 | 64.8 | 63.6 | 48.2 | 47.3 | 38.7 |

Lyophilised docetaxel ("lyo docetaxel") has a greater stability than the solutions of docetaxel in polysorbate 80 as it is used in the formulation currently available.
The solid composition of the present invention has a greater stability than docetaxel solutions in polysorbate 80 as they are used in the current formulation.

### Example 7

Docetaxel solid compositions obtained in Example 5 with different solubilising compositions were reconstituted. After obtaining a transparent solution in less than a minute, 2 minutes were allowed for the foam to settle and the solution obtained was injected into a container holding a perfusion solution, either normal saline or dextrose.

**TABLE IV**

| **Solubilising solution** | **Perfusion 0.475 mg/ml** | **Stability** |
|---|---|---|
| 4 ml/Solutol HS 15: Water (20:80) | Dextrose / saline | At least 6 hours / at least 6 hours |
| 4 ml/Solutol HS 15: Water (25:75) | Dextrose/saline | At least 6 hours/at least 6 hours |
| 4 ml/Solutol HS15:Water (30:70) | Dextrose/saline | At least 6 hours/ at least 6 hours |

where each vial containing the solid composition of lyophilised docetaxel 20mg was reconstituted with 4ml of solubilising composition.

### Example 8

Anhydrous docetaxel, purity 98.2%, in a quantity of 1.025g was placed into a 250ml container, and dissolved to a final volume of 80 ml of acetic acid.
Once dissolved, it was dosed in 1.6my of this solution in vials of 7ml. 48 doses of 20 mg each were obtained. Doses were pre-covered and taken to lyophilisation following a cycle of freezing stages of -60°C for 240 min, and lyophilisation at -5°C during 1500 min, at 5°C during 1500 min and final drying at 30°C during 2170 min. The lyophilisate resulted in a cake of a homogeneous form without adherence to the walls, with an apparent density of 0.0125 g/ml. The purity of docetaxel obtained by this process of lyophilisation was of 99.2% measured as a percentage area, detected by UV at 232 nanometers, in HPLC using a stainless steel column Waters Simmetry C18, of 4.6 mm x 15 cm, 5 microns and a mobile phase of acetonitrile : methanol : water (26:32:42, v:v:v) filtered and degassed.

### Example 9

Different vials of the solid composition of lyophilised docetaxel obtained in Example 8 were added by means of a needle 21G 1:1/2 solubilising solution constituted by water for injection and different concentrations of Solutol ® filtered by a membrane of 0.45µ. The result on the physical stability of the different experiences is shown in the following table, where the time spent by docetaxel to precipitate after mixing said solid composition and the solubilising composition can be seen on the last column.

**TABLE V**

| Docetaxel solid composition | Solubilising composition % Solutol ® | Volume | Dissolution time | Precipitation time |
|---|---|---|---|---|
| 1 vial (20 mg) | 20% | 2 ml | < 1 min | 1 hour |
| 1 vial (20 mg) | 20% | 2 ml | < 1 min | 2 hours |
| 1 vial (20 mg) | 20% | 2 ml | < 1 min | 1 hour |
| 1 vial (20 mg) | 20% | 2 ml | < 1 min | 2 hours |
| 1 vial (20 mg) | 20% | 2 ml | < 1 min | 3 hours |
| 1 vial (20 mg) | 20% | 2 ml | < 1 min | 8 hours |

### Example 10

Using a 21G 1:1 /2 needle, different vials of lyophilised docetaxel obtained in Example 8 were added with different quantities of aqueous solutions of Solutol 0 filtered by 0.45 micron membranes. The solution obtained by stirring in less than a minute was allowed to settle for about 5 minutes to decrease the quantity of foam and it was injected into dextrose solution 5% and normal saline (0.9%) to obtain docetaxel solutions of 0.5 mg/ml ready for perfusion. The results on the physical stability of the different experiences are shown in the following table as 'docetaxel precipitation time'

**TABLE VI**

| Docetaxel solid composition | Solubilising composition % Solutol | Volume | Injection in perfusion solution (0.5 mg/ml) | Precipitation time |
|---|---|---|---|---|
| 1 vial (20 mg) | 20% | 2 ml | Dextrose | 2 hours |
| 1 vial (20 mg) | 20% | 2 ml | Saline | 2 hours |
| 1 vial (20 mg) | 20% | 4 ml | Dextrose | 6 hours |
| 1 vial (20 mg) | 20% | 4 ml | Saline | 6 hours |
| 1 vial (20 mg) | 20% | 2 ml | Dextrose | 3 hours |
| 1 vial (20 mg) | 20% | 2 ml | Saline | 3 hours |
| 1 vial (20 mg) | 20% | 4 ml | Dextrose | 8 hours |
| 1 vial (20 mg) | 20% | 4 ml | Saline | 8 hours |
| 1 vial (20 mg) | 20% | 2 ml | Dextrose | 3 hours |
| 1 vial (20 mg) | 20% | 2 ml | Saline | 3 hours |
| 1 vial (20 mg) | 20% | 4 ml | Dextrose | 8 hours |
| 1 vial (20 mg) | 20% | 4 ml | Saline | 8 hours |

### Example 11

A 5% paclitaxel solution in acetic acid was prepared (free from acetic anhydride). Vials of 5 ml were dosed with this solution; 10mg of paclitaxel were obtained in each and they were subsequently frozen at -18°C for 10 h. Vials were lyophilised with an oil vacuum pump at ambient temperature for 10 h and dried at 35°C for 48 h, thus obtaining a paclitaxel solid composition as a cake.

Having thus specifically described and determined the nature of the present invention and the way of carrying it out, we hereby claim the following as of exclusive property and right:

## Claims

1. A pharmaceutical formulation of taxane to be administered to mammals, preferably humans, comprising two compositions which are combined prior to being administered, forming a transparent solution free from precipitates wherein:
a) the first of said compositions is a solid composition of lyophilised taxane and,
b) the second of said compositions is a solubilising composition of said solid composition of lyophilised taxane.

2. The formulation of claim 1, wherein said solid composition is free from tensoactives, oils, polymers, solubility enhancers, preservatives and excipients.

3. The formulation of claim 1, wherein said solid composition has an apparent density lower than 0.1 g/ml.

4. The formulation of claim 1, wherein said solid composition has an apparent density of 0.004 g/ml to 0.05 g/ml.

5. The formulation of claim 1, wherein said solid composition has an apparent density of 0.006 g/ml to 0.02 g/ml.

6. The formulation of claim 1, wherein said solid composition is soluble in an aqueous solution of 20% Solutol® HS 15 in less than a minute in the absence of an added organic solvent.

7. The formulation of claim 1, wherein said solid composition is chemically stable at 60°C for at least 28 days with a degradation lower than 1%.

8. The formulation of claim 1, wherein said solid composition is obtainable by lyophilisation in a solution comprising a lyophilisation organic solvent and a taxane.

9. The formulation of claim 1, wherein said solid composition has a residual lyophilisation organic solvent concentration lower than 8%.

10. The formulation of claim 1, wherein said solid composition has a residual lyophilisation organic solvent concentration lower than 3%.

11. The formulation of claim 8, wherein said lyophilisation organic solvent is selected from the group comprising dioxane, acetic acid, dymetylsuphoxide or a mixture thereof.

12. The formulation of claim 8, wherein said taxane is at a concentration of 0.1 to 50% in said solution.

13. The formulation of claim 8, wherein the concentration of said taxane is 0.1 to 6% in said solution. ,

14. The formulation of claim 8, wherein said solution comprises only said organic solvent and said taxane in the absence of tensoactives, oils, polymers, solubility enhancers, preservatives and excipients.

15. The formulation of claim 8, wherein said organic solvent is dioxane.

16. The formulation of claim 8, wherein said organic solvent is acetic acid.

17. The formulation according to any one of the preceding claims, wherein said taxane is selected from the group comprised by baccatin III derivatives, derivatives from 10-deacetilbaccatin III and conjugates, salts, hydrates and solvates thereof.

18. The formulation according to any one of the claims between 1 and 16, wherein said taxane is docetaxel, salts, hydrates or solvates thereof.

19. The formulation according to any of the claims between 1 and 16, wherein said taxane is paclitaxel, salts, hydrates or solvates thereof.

20. The formulation according to any of the claims between 1 and 19, wherein said solubilising composition comprises at least one tensoactive.

21. The formulation according to any of the claims between 1 and 19, wherein said solubilising composition comprises a polymeric tensoactive and water in the absence of organic solvent.

22. The formulation according to any of the claims from 1 to 21, wherein said solubilising composition comprises a tensoactive at a concentration of 1 % to 100%.

23. The formulation according to any claim from 1 to 21, wherein said solubilising composition comprises a tensoactive at a concentration of 5% to 40%.

24. The formulation according to any claim from 19 to 23, wherein said tensoactive is polymeric and selected from the group consisting of macrogol hydroxistearate, poloxamer, polyvinylpirrolidone or mixtures thereof.

25. The formulation according to any of the claims from 1 to 24 is free from polysorbate 80.

26. The formulation according to any of the claims from 1 to 24 is free from polyoxyethylated castor oil.

27. The formulation according to any of the claims from 19 to 23, **characterised by** said polymeric tensoactive is Solutol® HS 15.

28. The formulation according to claim 27, wherein said solubilising composition comprises Solutol HS 1 between 10 and 50% and water between 50% and 90% (P/P%).

29. The formulation according to any of the claims between 1 and 27, wherein said solubilising composition dissolves said solid composition at a concentration of at least 4mg/ml in the absence of precipitates for at least 2 hours.

30. The formulation according to any one of the preceding claims, wherein the solution obtained after combining said solid composition of taxane with said solubilising composition, when injected into normal saline solution or dextrose solution for perfusion, is transparent in the absence of *in situ* gelification and stable in the absence of precipitation for at least two hours.

31. A solid composition of taxane, suitable for the preparation of pharmaceutical formulations for mammals, in particular humans, wherein said solid composition by comprising said lyophilised taxane free from tensoactives, oils, polymers, solubility enhancers, preservatives and excipients.

32. The solid composition of claim 31, wherein the apparent density is from 0.001 g/ml to 0.1 g/ml.

33. The solid composition of claim 31, wherein the apparent density is from 0.004 g/ml to 0.05 g/ml.

34. The solid composition of claim 31, wherein the apparent density is 0.0067 g/ml to 0.02 g/ml.

35. The solid composition of claim 31, wherein said solid composition is soluble in aqueous solution of 20 % Solutol® in less than a minute in the absence of added organic solvent.

36. The solid composition of claim 31, wherein said solid composition is chemically stable at 60°C for at least 28 days with degradation lower than 1 %.

37. The solid composition of claim 31, wherein said solid composition has a residual organic solvent concentration of lyophilisation lower than 8%.

38. The solid composition of claim 31, wherein said solid composition has a residual lyophilisation organic solvent concentration lower than 3%.

39. The solid composition of claim 31, wherein said solid composition is obtainable by the lyophilisation of a solution comprising an organic solvent of lyophilisation and a taxane.

40. The solid composition of claim 39 wherein said lyophilisation organic solvent is selected from the group consisting of dioxane, acetic acid, diletylsulphoxide or a mixture thereof.

41. The solid composition of claim 39, wherein said lyophilisation organic solvent is dioxane.

42. The solid composition of claim 39, wherein said lyophilisation organic solvent is acetic acid.

43. The solid composition of claim 39, wherein said taxane is present in said solution at a concentration of 0.1 to 50%.

44. The solid composition of claim 39 wherein said taxane is present in said solution at a concentration of 0.1 to 6%.

45. The solid composition of claim 39 wherein said solution comprises only said lyophilisation organic solvent and said taxane in the absence of tensoactives, oils, polymers, solubility enhancers, preservatives and excipients.

46. The solid composition from claims 31 to 45 wherein said taxane is selected from the group comprised by baccatin III derivatives, 10-deacetylbaccatin III derivatives and conjugates, salts, hydrates and solvates thereof.

47. The solid composition from claims 31 to 45 wherein said taxane is docetaxel, its salts, its hydrates or solvates.

48. The solid composition of claims 31 to 45 wherein said taxane is paclitaxel, salts, hydrates or solvates thereof.

49. A procedure for the preparation of a solid composition of taxane, suitable to prepare pharmaceutical formulations for mammals, mainly humans, comprising the following steps:
a) dissolving said taxane in lyophilisation organic solvent in the absence of tensoactives, oils, polymers, solubility enhancers, preservatives and excipients.
b) lyophilising
c) drying (optionally).

50. The procedure of claim 49 wherein said procedure also comprising a sterilisation stage.

51. The procedure of claim 50 wherein said stage of sterilisation comprises a sterilizing filtration of the solution obtained in step a) of claim 49.

52. The procedure of claim 49 **characterised by** said taxane is selected from the group comprised by baccatin III derivatives, 10-deacetylbaccatin III derivatives, conjugates, salts, hydrates and solvates thereof.

53. The procedure of claim 49 wherein said taxane is docetaxel, its salts, its hydrates and solvates.

54. The procedure of claim 49 wherein said taxane is paclitaxel, its salts, its hydrates or solvates.

55. A solubilising composition of solid compositions of taxanes suitable to prepare injectable pharmaceutical formulations for parenteral infusion into mammals, mainly humans, comprising at least one tensoactive.

56. The solubilising composition of claim 55 wherein said solubilising composition comprises a polymeric non-ionic tensoactive and water in the absence of an organic solvent.

57. The solubilising composition of any of the claims from 55 to 56 wherein said solubilising composition comprises a polymeric non-ionic tensoactive at a concentration of 0.1 % to 50%.

58. The solubilising composition of any of the claims 55 to 56 wherein said solubilizing composition comprises a polymeric non-ionic tensoactive at a concentration of 10% to 40%.

59. The solubilising composition of any claim from 55 to 58 wherein said tensoactive is Solutol® HS 15.

60. A pharmaceutical perfusion solution containing less than 1 mg/ml of taxane in normal saline solution or dextrose solution, comprising also Solutol®, basically free from organic solvent, other tensoactives, oils, other polymers, solubility enhancers, preservatives and excipients.

61. The solution of claim 60, wherein said taxane is docetaxel.

62. A kit for the formulation of injectable taxane, comprising a first container containing a solid composition of lyophilised taxane as claimed in any of the claims from 31 to 48; a second container containing a solubilising composition of said solid composition of taxane, and a syringe.

63. The kit according to claim 62 wherein said syringe being prefilled, and comprises said first container and said second container.

64. A kit for the preparation of an injectable formulation of taxane, suitable to prepare parenteral infusion solutions for mammals, preferably humans, comprising a solid composition of said lyophilised taxane as any of the claims between 31 and 48 a solubilising composition of said solid composition of said taxane as the one described in any of the claims from 55 to 59; a syringe to mix said solubilising composition with said solid composition of said taxane and to obtain, therefore, a transparent and stable solution of taxane at a concentration of at least 4 mg/ml to be injected into the parenteral infusion bag free from precipitation for at least 2 hours.
